(19) Europäisches Patentamt / European Patent Office / Office européen des brevets

(11) EP 3 782 976 B1

(12) **EUROPEAN PATENT SPECIFICATION**

(45) Date of publication and mention of the grant of the patent:
**18.10.2023 Bulletin 2023/42**

(21) Application number: **19192802.7**

(22) Date of filing: **21.08.2019**

(51) International Patent Classification (IPC):
***C07C 67/08*** *(2006.01)* ***C07C 69/593*** *(2006.01)*
***C07C 69/602*** *(2006.01)* ***C07C 69/44*** *(2006.01)*
***C07C 51/377*** *(2006.01)* ***C07C 55/14*** *(2006.01)*

(52) Cooperative Patent Classification (CPC):
(C-Sets available)
**C07C 67/08** (Cont.)

(54) **SUSTAINABLE PROCESS FOR PRODUCING MUCONIC, HEXENEDIOIC AND ADIPIC ACID (AND THEIR ESTERS) FROM ALDARIC ACIDS BY HETEROGENEOUS CATALYSIS**

NACHHALTIGES VERFAHREN ZUR HERSTELLUNG VON MUCON-, HEXANDION- UND ADIPINSÄURE (UND IHREN ESTERN) AUS ALDARSÄUREN DURCH HETEROGENE KATALYSE

PROCÉDÉ DURABLE DE PRODUCTION D'ACIDE MUCONIQUE, HEXÈNEDIOÏQUE ET ADIPIQUE (ET LEURS ESTERS) À PARTIR D'ACIDES ALDARIQUES PAR CATALYSE HÉTÉROGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.02.2021 Bulletin 2021/08**

(73) Proprietor: **Kemijski Institut**
**1000 Ljubljana (SI)**

(72) Inventors:
- **HOCEVAR, Brigita**
  **1303 Zagradec (SI)**
- **LIKOZAR, Blaz**
  **4000 Kranj (SI)**
- **GRILC, Miha**
  **1291 Skofljica (SI)**

(74) Representative: **Weickmann & Weickmann PartmbB**
**Postfach 860 820**
**81635 München (DE)**

(56) References cited:
**WO-A1-2015/189481** **WO-A1-2016/032403**
**WO-A1-2017/207875** **KR-A- 20170 016 227**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**C07C 67/08, C07C 69/44;**
**C07C 67/08, C07C 69/593;**
**C07C 67/08, C07C 69/602**

## Description

The present invention relates to the use of a heterogeneous $Re^0$ catalyst for the conversion of biobased aldaric acids to muconic, hexenedioic acid, adipic acid and their esters in a batch stirred slurry reactor or continuous fixed-bed reactor. The process comprises conversion of aldaric acid (preferably mucic or glucaric acid) into muconic, hexenedioic or adipic acid and their esters. Aldaric acid in the concentration range of 0.01 - 50 wt% (with regards to the total mass of the liquid phase) is dissolved in the short chain primary or secondary alcohol (e.g. methanol) and a Re-based solid catalyst (0.1 - 400 wt% based on the mass of aldaric acid) is added to the reaction mixture, which is proceeded at the temperature within 80 - 250 °C under inert ($N_2$) or hydrogen atmosphere for 1 - 340 h to yield esters of muconic, hexenedioic acid, adipic acid. Further hydrolysis of ester converts products into their free carboxylic acid form.

## DESCRIPTION

### FIELD OF THE INVENTION

The present disclosure relates generally to a novel catalytic process as defined in claim 1 for production of muconic and adipic acid and their esters from bio-based aldaric acids in an alcohol over a solid catalyst in an inert atmosphere (no reductive gas required). Catalyst required for this transformation consists of rhenium (Re) in its metallic form and a solid support (e.g. carbon, alumina, silica), thus being insoluble in a reaction medium.

This is the first report of an active solid catalyst for dehydroxylation of aldaric acids, which is a major benefit, as it is easy to separate from the products by a simple filtration, sedimentation or allows its use in a continuous (fixed-bed) operation.

This invention is also the first to utilize a heterogeneous (solid) catalyst and at the same time avoids the use of any corrosive reactants (e.g. HBr) and the necessity to use gaseous reductive agent (e.g. $H_2$).

### BACKGROUND OF THE INVENTION

The depletion of fossil resources like crude oil, natural gas, and coal with a negative impact on climate changes has inspired academic and industrial researchers to find alternative energy carriers and source of commodity chemicals based on renewable raw materials. Adipic acid is produced in 3.7 billion kg annually; market exceeds 7 billion UDS/year, where more than 60 % of the total production is used as a monomer for nylon 66 production (polycondensation reaction with hexamethylene diamine). Currently adipic acid is produced from fossil resources (crude-oil based cyclohexane) in the process where a corrosive nitric acid is used and NOx emissions are formed. Highly exothermic oxidation step needs to be operated at very low conversion to run safely, which increases the cost of the overall process.

It is already known (PCT/FI2015/050432, same as WO/2015/189481), that Re-based homogeneous catalyst (methyl trioxo rhenium, $CH_3ReO3$, CAS number 70197-13-6) dissolved in alcohol can convert aldaric acids into muconic acids and furans under elevated hydrogen pressure between 1-20 bar.

It was also reported (Larson et al., J. Am. Chem. Soc. 2017, 139, 40, 14001-14004 (doi:10.1021/jacs.7b07801), Raju et al., ACS Catal. 2015, 5, 1, 281-300 (doi:10.1021/cs501511x), Shiramizo and Toste, Angew. Chem. Int. Ed., Vol. 52, 49, 12905-12909 (doi:10.1002/anie.201307564)) that aldaric acids can be dehydroxylated into muconic acids in alcohols and dissolved homogeneous Re-based catalysts ($KReO_4$, CAS number 10466-65-6, $HReO_4$, CAS number 13768-11-1, CHsReOs, CAS number 70197-13-6) in oxidative atmosphere. Selective dehydroxylation of aldaric acids towards muconic acid is presented in the above-mentioned patent. Reaction is performed over a homogeneous methyloxorhenium catalyst at the temperature of 100 °C, using light alcohols as a solvent. At elevated temperature (200 °C) reaction is shifted towards the furan route, forming furoic acid. Reaction is performed under the reductive atmosphere of molecular hydrogen. Even though the solvent and $H_2$ can be recycled from the reaction mixture and re-used, the homogeneous catalyst is difficult to be separated from the product. The highest reported yield of muconic acid ester was 21.4 %.

Similarly, another patent (WO 2010/0144862) describes the synthesis of adipic acid from glucaric (aldaric) acid over a homogeneous methyloxorhenium catalyst (MTO) in alcohol as a solvent, but in the presence of para-toluenesulfonic acid. The process is subdivided into three steps, where in the first step dehydroxylation of aldaric acids toward muconic acid esters is performed over MTO catalyst in alcohol; muconic acid ester is further hydrogenated to adipic acid ester over Pd catalyst in hydrogen atmosphere. Adipic aicd esters are further converted into adipic acid in ethanol/water solvent in the presence of NaOH and concentrated hydrochloric acid. Exact yield of produced adipic acid was not revealed.

Similar to the presented patents few other studies tackled with selective dehydroxylation of aldaric acids towards muconic acid, adipic acid and their esters over rhenium catalysts. Zhang research group (Li et al, Angew. Chem. Int. Ed., Vol. 53, 16, 4200-4204 (DOI: 10.1002/anie.201310991)) reported highly efficient conversion of mucic acid towards adipic aicds over methyloxorhenium catalyst (MTO) or $Re_2O_7$ in 3-pentanol. High yield (99 %) of adipic acid ester(s)

was reached in a one-pot reaction using a combination of Re and Pt/C catalyst in the presence of paratoluenesulfonic acid under air flow; however lower yield was reached under nitrogen ($N_2$) flow.

Larson (J. Am. Chem. Soc. 2017, 139, 40, 14001-14004 (DOI: 10.1021/jacs.7b07801)) reported efficient conversion of glucose derivatives into adipates over oxo-Re (homogeneous) catalysts.MeReOs and $KReO_4$ were used in a combination of Pd/C catalyst for conversion of aldaric acid lactones into adipic acid in alcohols or water as solvents. 72 % yield of adipic acid was obtained under the aqueous conditions after 15 h of reaction performing at 150 C. Hydrogen was used as a terminal reductant in a gaseous phase at 5 bar.

Shiramizu (Angew. Chem. Int. Ed., Vol. 52, 49, 12905-12909 (doi: 10.1002/anie.201307564)) tested different oxo-rhenium catalysts (CHsReOs, $HReO_4$ and $KReO_4$) for conversion of mucic acid into muconic acid and adipates in different solvents (3-pentanol or n-butanol), and gaseous phase ($H_2$ or $N_2$).

The use of heterogeneous Re catalysts were mentioned only for dehydroxylation of simple diols into olefins and/or alkanes at high yields, since the use for sugar acids, specifically for aldaric acid was not reported yet. Tomishige research group (Ota et al., Angew. Chem. Int. Ed., Vol. 54, 6, 1897-1900 (DOI:10.1002/anie.201410352), Ota et al., ACS Catal. 2016, 6, 5, 3213-3226 (DOI:10.1021/acscatal.6b00491), Tazawa et al., ACS Catal. 2016, 6, 10, 6393-6397 (DOI-10.1021/acscatal.6b01864)) developed efficient $CeO_2$ supported ReOx catalyst for dehydroxylation of vicinal diols. Other supports (e.g. C, $Al_2O_3$, $ZrO_2$,$TiO_2$, MgO, CaO, $La_2O_3$, $Y_2O_3$) showed minor activity for selected dehydroxylation of vicinal diols and only minor conversion (< 5 %) of reactant (1,4-anhydroerythritol). The addition of gold nanoparticles showed promoting effect on the dehydroxylation reaction.

Rennovia patented the process (US 2010/0317823) where adipic acid is formed from aldaric acids in glacial acetic acid, acidified with HBr, under elevated hydrogen pressure over noble metals such as Rh, Pt, Pd and Ru on various supports ($SiO_2$ and $TiO_2$). Their invention presents preparation of glucaric acid from glucose in the first step and further dehydroxylation of glucatric acid into adipic acid. Only the second reaction (conversion of glucaric aicd into adipic acid) is relevant for this case. Reaction is performed in glacial acetic acid in the presence of HBr over different silica or titania supported metal catalyst. However, Re was not tested in the proposed process. Except of the same reactant and product, our proposed process is completely different from the one patented by Rennovia.

Patent application KR 2017/0016227 describes the preparation of dibuptyl adipate by first oxidizing galactose to the respective aldaric acid and then heating in the presence of a rhenium oxide catalyst in butanol. Thus, in contrast to the present invention it uses a rhenium oxide catalyst.

International application WO 2016/032403 describes a procedure for the conversion of mucic acid to muconic acid or adipic acid. The reaction is carried out in the presence of a rhenium-based catalyst. In Tables 1-4, various rhenium catalysts are suggested, all of which have in common that they contain rhenium oxides, i.e. rhenium having higher oxidation states. There is no hint as to the use of metallic rhenium supported on a solid carrier.

International application WO 2017/207875 is drawn to producing muconic acid from aldaric acids. The conversion is carried out in butanol in the presence of a rhenium catalyst. In the examples, ammonium perrhenate is used. Rhenium is thus present in a high oxidation state. There is no hint as to using a heterogenous catalyst where metallic rhenium is supported on a solid carrier.

## SUMMARY OF THE INVENTION

Present invention solves the problems outlined above and features a sustainable process where adipic acid (and their esters and non-saturated derivatives) is produced from bio-based aldaric acids (formed by oxidation of glucose or other sugar-related compounds) by a selective removal of hydroxyl groups. Our process is the first to selectively dehydroxylate aldaric acids over heterogeneous catalysts and without the use of gaseous hydrogen or corrosive HBr reactants. As reported in the literature and patents, the most efficient catalyst for dehydroxylation of vicinal diols are homogeneous catalysts, specifically oxo-rhenium complexes. However, a homogeneous catalyst cannot be easily recycled from the reaction mixture. Therefore we have developed a process as defined in the appended claims, where a solid Re in metal form is supported on solid alumina, silica, titania, zirconia, carbon or other support ($CeO_2$, $MgO_2$) and effectively converts aldaric acids into muconic acid. To yield non-saturated derivatives of adipic acid (e.g. mucconic acid) dehydroxylation can take place without hydrogen in an inert atmosphere. After dehydroxylation of aldaric acids, muconic acid is formed, which is efficiently hydrogenated with molecular hydrogen in the second step where Re or Re/Pd heterogeneous catalysts are used. Other hydrogenation metals (e.g. Pt) can also efficiently promote complete hydrogenation of double bonds. Heterogeneous catalyst(s) can be easily separated from reaction mixture by filtration and reused.

Catalytic process can take place in a continuous reactor with fixed catalyst bed, where a dissolved aldaric acid flows through it. Catalytic process can also take place in a batch, semi-batch or continuous stirred tank (slurry) reactor with dispersed catalyst or a catalyst in a rotating basket where the catalyst is simply removed from the slurry by filtration or sedimentation.

The highest yield (62.4 mol%) of 2,4-hexadienedioic acid dimethyl ester, presented as (2) in Figure 1 was achieved over Re/C under inert atmosphere, while the highest yield of dimethyl adipate (hexanedioic acid dimethyl ester-

presented as (7) in Figure 1) was obtained with a combination of Re/C and Pd/C.

## BRIEF DESCRIPTION OF THE FIGURES

Figure 1: Proposed reaction scheme of an aldaric acid conversion into (1) Mucic acid dimethyl ester, (2) 2,4-hexadienedioic acid dimethyl ester (3) 2-hexenedioic acid dimethyl ester (E), (4) 2-hexenedioic acid dimethyl ester (Z), (5) 3-hexenedioic acid dimethyl ester (E), (6) 3-hexenedioic acid dimethyl ester (Z), (7) Hexanedioic acid dimethyl ester, and (8) Hexanedioic acid (adipic acid).

Figure 2: Reactor set-up. A high-pressure stirred batch reactor.

Figure 3: GC-MS/FID chromatogram and (EI) mass spectra of obtained products.
3a: Typical GC-MS/FID chromatogram of obtained products; over Re/C in MeOH under $H_2$ (and $N_2$) atmosphere.
3b: (EI) mass spectra of 2,4-hexadienedioic acid dimethyl detected by MS (upper spectrum) and agreement with NIST 17 library (lower spectrum).
3c: (EI) mass spectra of 2-hexenedioic acid dimethyl ester (E) detected by MS (upper spectrum) and agreement with NIST 17 library (lower spectrum).
3d: (EI) mass spectra of 2-hexenedioic acid dimethyl ester (Z) detected by MS (upper spectrum) and agreement with NIST 17 library (lower spectrum).
3e: (EI) mass spectra of 3-hexenedioic acid dimethyl ester (E) detected by MS (upper spectrum) and agreement with NIST 17 library (lower spectrum).
3f: (EI) mass spectra of 3-hexenedioic acid dimethyl ester (Z) detected by MS (upper spectrum) and agreement with NIST 17 library (lower spectrum).
3g: (EI) mass spectra of Hexanedioic acid dimethyl ester (a.k.a. dimethyl adipate) detected by MS (upper spectrum) and agreement with NIST 17 library (lower spectrum).

## DETAILED DESCRIPTION OF THE INVENTION

The present invention relates to the sustainable and green production of nylon precursors (muconic acid, adipic acid and their esters) over a heterogeneous (solid) Re catalyst in a batch or continuous reactor. The invented process can eventually compete to or substitute current industrial production of these chemicals from crude oil, since it uses heterogeneous (solid, insoluble) catalysts, which are easily separated from reaction mixture after use or can be fixed in a continuous fixed-bed reactor. Heterogeneous catalytic system offers considerable advantage in terms of cost reduction of downstream processing. Moreover, in the proposed process greenhouse gas emissions are omitted, as well as the use of hazardous (e.g. toxic, corrosive) chemicals.

The present invention comprises the step of converting a starting material, selected from aldaric acids (named hexaric acids): (allaric (meso), formerly allo-mucic; galactaric (meso), formerly mucic; D- and L-glucaric (L-and D-gularic), formerly D-and L-gluco-saccharic; D- and L-mannaric, formerly D- and L-manno-saccharic; D-and L-idaric, formerly D- and L-ido-saccharic; and D- and L-altraric(D- and L-talaric), formerly D- and L-talo-mucic acid); preferably biobased lactose based mucic acid from or glucose originating glucaric acid. It has been found that the amount of a starting material has to be in the range between 0.1 to 20 wt%, preferably from 0.1 to 5 wt% due to low solubility at room temperature in any kind of solvent. The solubility increases with the temperature, thus running experiments at elevated temperature is preferable.

The starting material is dissolved in the solvent, selected from short chain primary or secondary alcohols, also being an active compound for esterification of the starting material. Solvent is selected among methanol, ethanol, 1- or 2- propanol, 1- or 2-butanol, or higher alcohols; however methanol is preferably used due to the low price, highest activity and lowest boiling point which delivers many benefits as it facilitates downstream and separation processes. Alcohol serves as a source of hydrogen if needed in the hydrogenation step, thus no additional reductive atmosphere (e.g. $H_2$) is required. Methanol, adsorbed on the catalyst surface, is converted into formaldehyde, further rapidly converted into aldol condensation products. Hydrogen separated from methanol participates in hydrogenation process, where double bonds are completely or partially saturated. Results showed that methanol delivers the highest yields of (2) - (7) products; this is 92.0 mol%, while larger and branched alkyl groups deliver slightly lower yields. Obtained yield (92.0 mol%) is to our best knowledge the highest reported yield obtained over heterogeneous catalyst so far.

Catalyst, used in the present process is selected from supported Rhenium metal catalysts. Rhenium can be supported on neutral or acidic support, which is selected from carbon (C), alumina ($Al_2O_3$), silica ($SiO_2$), titania ($TiO_2$),

and similar oxides listed in the claims. Mixed oxides in different ratios can also be used as a support. Preferably neutral support is selected to avoid formation of side products; however, the most preferable is carbon support due to its inert surface and large surface area. Considerably lower activity was noticed running the reaction over alumina and silica, 6.0 and 6.5 mol%, respectively.

Re is present in oxidation state 0 on the catalyst surface. $Re^0$, where only Re metal nanoparticles are at the carbon surface and oxygen is removed, is most active and selective for dehydroxylation reaction. To ensure the metallic form on the catalyst surface, pre-reduction step is needed.

Prior to the reaction, rhenium catalyst is reduced in a furnace at the temperature between 250 and 550 °C, preferably 350 and 450 °C, in a reductive atmosphere, preferable in a flow of hydrogen. The reduction step is completed within a reasonable time (1-12 h), preferable after 4 h.

A combination of two metals on the same support can be used, whereas the amount of Re metal according to other metal is in abundance. A combination of dehydroxylation (Re) catalyst and hydrogenation one (Pd or Pt) is preferable; still the amount of rhenium presents majority of metal content on the support (ranging from 1 - 20 wt% relative to the support). The amount of hydrogenation metal, preferably Pd or Pt is in the range 0.1 - 5 wt% corresponding to the support.

Using a stirred batch reactor reaction is performed under the low pressure of inert atmosphere or higher pressure (10 - 70 bar) of reductive (e.g. $H_2$) atmosphere in order to completely saturate the 2,4-hexadienedioic acid (dimethyl ester) into hexanedioic acid (dimethyl ester). Performing reaction in the continuous (fixed-bed) reactor no gas phase introduction is required.

Stirring speed in a batch reactor is in the range from 100 -10 000 rpm, preferably from 300 - 2000 rpm and most preferably from 600 - 1000 rpm while the reaction time required for full conversion is in the range of 1 to 340 h, preferably from 24 to 100 h, and the most preferably 48 - 72h, depending on the reaction conditions.

The process disclosed here is carried out at a temperature of from 80 to 250 °C, preferably from 100 - 200 °C and most preferably from 120 - 170 °C. A preferred combination of reaction conditions is a temperature from 120 - 170 °C, stirring speed of 600 - 1000 rpm typically for 48 - 72h.

The reaction may be carried out in any high pressure reactor or closed autoclave, since the pressure increases with the temperature due to the low boiling point of solvent; however stainless steel reactors (batch/slurry or continuous/fixed-bed) are in particular suitable.

The examples described herein present typical catalytic conversion of aldaric acid in a stainless steel batch reactor under the standard operation conditions. A combination of a heterogeneous Re based catalyst, cheap non-hazardous solvent (methanol), biobased feedstock (aldaric acid) and low reaction temperature (120 °C) is the novelty in the present process for production of muconic acid, adipic acid and their esters. More specifically, the present invention was carried out at a temperature between 120 and 175 °C, most preferably at 120 °C under inert ($N_2$) atmosphere at low pressure over Re supported on neutral carbon support. Table 1 and Table 2 summarize obtained experimental results in accordance with the present invention.

## EXAMPLES

The following examples are included to further illustrate various embodiments of the presently disclosed subject matter. However, those of ordinary skill in the art should, in light of the present disclosure, appreciate that many changes can be made in specific embodiments which are disclosed and still obtain like or similar results without departing from the scope of the presently disclosed subject matter

### Materials used

In the next examples, the following starting materials were used:
Nitrogen (5.0, Messer, Bad Soden am Taunus, Germany), hydrogen (5.0, Messer, Bad Soden am Taunus, Germany), rhenium(III) chloride (Sigma Aldrich), activated carbon (C3345, Sigma Aldrich), commercially available 5% Re/C (Riogen Inc., New Jersey, USA), mucic acid (Sigma Aldrich, >97 %), glucaric acid (Sigma Aldrich, >98 %), methanol (Sigma-Aldrich, >99.9%), 2-propanol (Sigma-Aldrich, >99.5%).

All these materials were used as received from the suppliers, i.e., without any additional purification.

## EXAMPLE 1

### Catalysts preparation

The preparation of the Re/C catalysts has involved the impregnation of activated carbon using the incipient wetness impregnation method with solution of rhenium chloride. Samples were prepared by slowly adding 1.8 mL of 0.1

M aqueous $ReCl_3$ solution to 1 g of activated carbon. The sample was heated-up to 50 °C and stirred vigorously until the suspension turned into slurry. Then samples were dried overnight at 110 °C and reduced at 400 °C under a flow of pure hydrogen for 3 hours.

## EXAMPLE 2

### Catalytic dehydroxylation of mucic acid over Re/C in MeOH under $H_2$ atmosphere

Dehydroxylation tests were studied in a high pressure high temperature batch autoclave (volume of 250 mL, Amar Equipment Pvt. Ltd., India). Temperature in the reactor was followed by thermocouple, regulated by heating coat and inner cooling system. Reactor system was equipped with a pressure gauge, rapture disc, gas release valve and liquid phase sampling line that allows the sampling of the liquid phase during the process. In a typical experiment, 0.2 g of biobased mucic acid, 0.4 g of carbon supported Re catalyst and 95.0 g of MeOH were weighted into the autoclave vessel, attached to the housing and sealed. The reactor was first purged with $N_2$ three times to remove any residual of oxidizing atmosphere and twice with $H_2$, and then pressurized with $H_2$ to 5 bar. The stirrer speed was set to 600 rpm; the final temperature was set to 120 °C and reached in 25 minutes. Once the temperature reached the plateau, the reaction took place for another 72 hours. Liquid samples were taken during the reaction time in order to determine the yield of desired products by gas chromatography and mass spectrometry (GCMS-QP 2010 Ultra, Shimadzu, Kyoto, Japan). After the reaction was completed, the reactor was cooled-down to the room temperature. Cooled gaseous phase was analyzed three times by micro GC (490 Micro GC System, Agilent, Santa Clara, CA, USA) and then decompressed. Obtained final reaction mixture was analyzed by GCMS. Under tested reaction conditions the obtained final yield of desired products (completely dehydroxylated dimethyl esters of mucic acid with one two or none double bonds; Figure 1, products (2-7)) was 83.2 mol%; specifically 1.5 mol% of 2-hexenedioic dimethyl ester (Z), 25.1 mol% of hexanedioic acid dimethyl ester, 8.6 mol% of 3-hexenedioic acid dimethyl ester (Z), 23.8 mol% of 3-hexenedioic acid dimethyl ester (E), 24.0 mol% of 2-hexenedioic acid dimethyl ester (E) and 0.2 mol% of 2,4-hexadienedioic acid dimethyl ester after 72 hour of reaction, and 1.1, 4.4, 6.7, 21.7, 12.9, and 1.3 mol% after 36h, respectively. After 60 h of reaction time solely hydrogenation of double bonds proceeded, dehydroxylation reaction was complete. Detecting low concentration of (2) means the dehydroxylation reaction proceeds slower than hydrogenation. Hydrogenation is known to be fast, if sufficient hydrogen is present and consequently adsorbed on the catalyst surface. The pressure reached 9.4 bar at the temperature of plateau.

### Analytic methods used for gaseous and liquid phase analysis.

Final cooled gaseous phase formed during the reaction was analyzed by micro GC (490 Micro GC System, Agilent, USA) equipped with two different columns (MS5A - Molecular Sieve Packed Column, length of 10 m- and PPU - ParaPLOT column; each length of 10 m). Compounds separated by MS5A and ParaPLOT columns were detected by TCD (thermal conductivity) detector. The injector temperature was maintained at 363 K and column temperature at 373 K during the analysis.

Liquid samples collected during the reaction time and final reaction mixture were directly analyzed by GCMS without any preparation of samples. GCMS was equipped with a nonpolar column (ZebronTM ZB-5MS, length 60m, diameter 0.25 mm, film thickness 0.25 μm). Compounds were identified by mass spectrometry (MS; fragments were scanned in the range from 35 to 600 *m/z* and were compared to the NIST 17 and FFNSC libraries) and quantified by FID (flame ionization) detector. Injection temperature was 333 K, injection volume 0.5 μL and split ratio 5. The initial column temperature was 333 K kept on it for 5.5 min, then increased to 563 K with a heating rate of 20 K $min^{-1}$ and kept for additional 8 min. The FID temperature was maintained at 563 K. The following retention times belongs to the suitable compounds: 2-hexenedioic dimethyl ester (Z) RT = 13.89 min, hexanedioic acid dimethyl ester RT = 13.98 min, 3-hexenedioic acid dimethyl ester (Z) RT = 14.02 min, 3-hexenedioic acid dimethyl ester (E) RT = 14.10 min, 2-hexenedioic acid dimethyl ester (E) RT = 14.28 min, 2,4-hexadienedioic acid dimethyl ester RT = 14.49 min.

## EXAMPLE 3

### Catalytic dehydroxylation of mucic acid over $Re/Al_2O_3$ in MeOH under $H_2$ atmosphere

Dehydroxylation reaction were studied in (same as Example 2) high pressure three phase batch reactor (volume of 250 mL, Amar Equipment Pvt. Ltd., India). In a typical experiment 0.2 g of reactant (mucic acid), 0.4 g of Re catalyst supported on acidic alumina support and 95.0 g of MeOH were weighted into the reactor vessel, placed in the reactor housing and sealed. Reaction temperature was maintained at 120 °C by heating jacket and inner cooling system. Reaction was performed under 5 bar of initial hydrogen pressure and it reached 9.4 bar at the plateau temperature (120 °C).

During the reaction time liquid samples were collected in appropriate time intervals and were further analyzed by GCMS according to the method presented in Example 2. Final cooled gas phase was analyzed by micro GC using same method as presented in Example 2. The yield of desired products (Figure 1 (2-7)) obtained over acidic support was significantly lower (6.0 mol%) if compared to the Re catalyst supported on carbon.

## EXAMPLE 4

### Catalytic dehydroxylation reaction over Re/C in MeOH under $N_2$ atmosphere

Dehydroxylation reaction was (same as Example 2 and 3) performed in a three phase high pressure batch reactor (250 mL, Amar Equipment Pvt. Ltd., India). Same ratio of reactant, catalyst and solvent (methanol) was used as in Example 2 and Example 3; 0.2 g of mucic acid, 0.4 g of Re/C catalyst and 95.0 g of solvent (methanol), which were placed into the reactor vessel, closed and sealed. Further, the headspace of reactor volume was flushed three times with nitrogen and finally pressurized with $N_2$ to the desired pressure (initial 5 bar, reached 9.4 bar at the plateau temperature). Collected liquid samples and cooled final gas phase were analyzed by a GCMS and a $\mu$GC, respectively, by following the same methods as in Example 2 and Example 3. The obtained yield of desired products was slightly higher under the inert atmosphere compared to the example under hydrogen atmosphere and generally only dehydroxylation reaction proceeds. The yield of 2,4-hexadienedioic acid dimethyl ester reached 62.4 mol%, the yield of (3) - (6) reached 27.9 and 1.7 mol% was (7). The summarized yield was 92.0 mol%. Under inert atmosphere dehydroxylation process proceeds first and much faster as hydrogenation. Surprisingly, there is no need of additional hydrogen source for dehydroxylation to proceed, while MeOH presents the hydrogen source. MeOH is adsorbed on the catalyst surface and was converted into aldehyde, while released hydrogen is then involved in the dehydroxylation of aldaric acid.

## EXAMPLE 5

### Catalytic dehydroxylation of mucic acid over Re/C in 2-propanol under the inert $N_2$ atmosphere

Dehydroxylation process was performed in a batch three phase stirred reactor (volume of 75 mL (Series 5000 Multiple Reactor System, Parr Instrument Company, Moline, IL, USA), equipped with a magnetic stirrer, heating system and a sampling line for liquid samples collection during the reaction time at elevated pressure. In a typical experiment 0.075 g of mucic acid, 0.150 g of Re/C catalyst and 35.6 g of 2-propanol was weighted into reaction vessel, placed to the reactor housing and sealed. Before heating up reactor headspace was flushed three times with nitrogen to remove any oxygen from a headspace. Reaction was performed under inert nitrogen atmosphere starting at the initial 5 bar of $N_2$, eventually increased to the 7.4 bar when the temperature reached the plateau (120 °C). 2-Propanol solvent caused the formation of 2,4-hexadienedioic acid diisopropyl ester with the yield of 75.2 mol% after 48 h and 85.7 mol% after 72h. Only minor concentrations of (3) - (7) products were detected. 2-propanol as a secondary alcohol forms ketone after dehydration.

## EXAMPLE 6

### Testing the long term stability and activity of the catalyst

The stability and activity of the catalyst was studied in four consecutive catalytic tests, each performed for 72h as in a typical example. The reaction mixture was cooled down after the reaction was finished and gaseous phase was released from reactor headspace. The catalyst deposited at the bottom of the reactor after few hours, and only liquid reaction mixture was removed from the reactor. Solid catalyst was further washed with reaction solvent (methanol) and centrifuged. At least 5 cycles with pure methanol washing, centrifuging and removing the liquid phase was repeated to completely remove all traces of unreacted aldaric acid, intermediates and products (confirmed by GCMS). Firstly catalyst was reduced only before first run. Results showed its activity for two experiments (duration of 72 h), later immediately dropped for 50 %. If a catalyst is reduced after each run, its activity is retained for few more samples. Catalyst reduction proceeded in the tube furnace at 400 °C for 3 h under the constant hydrogen flow of 200 mL $min^{-1}$. The ratio between reactant, catalyst and solvent was kept constant in all recycling tests. The yield of products was calculated and the loss of catalyst activity during the recycling test was followed.

Catalyst activity and stability was tested in long term catalytic test for 336 h. After 270h 2,4-hexadienedioic acid dimethyl ester was completely converted into hexadienedioic acid dimethyl esters ((3) - (6)) and only in minor to dimethyl adipate (7). Furthermore soon after (2) was completely converted, the equilibration between (3), (4), (5), and (6) was established. The catalyst is active for a long term experiments, which makes it suitable for a continuous reactions.

*Yield calculation*

Calibration curves of external standards (dehydroxylated and esterified products (2-7)) were prepared and measured by GCMS according to the same method as in Example 2-5. The yield of each compound was calculated in mol% as:

$$Y_i(mol\,\%) = \frac{c_i}{c_M} x100$$

| Catalyst | Gas phase | Yield (mol%) **(2)** 2,4-hexadienedioic acid dimethyl ester 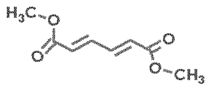 | Yield (mol%) **(3)** 2-hexenedioic acid dimethyl ester (E) 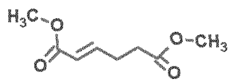 | Yield (mol%) **(4)** 2-hexenedioic acid dimethyl ester (Z) 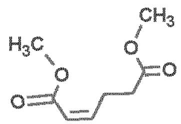 | Yield (mol%) **(5)** 3-hexenedioic acid dimethyl ester (E) 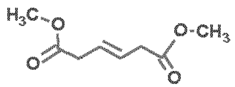 | Yield (mol%) **(6)** 3-hexenedioic acid dimethyl ester (Z) 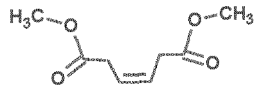 | Yield (mol%) **(7)** Hex-anedioic acid dimethyl ester 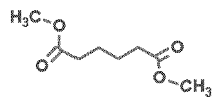 | Yield (mol%) **PRODUCTS (2) - (7)** |
|---|---|---|---|---|---|---|---|---|
| Re/C | Hz | 0.2 | 24.0 | 1.5 | 23.8 | 8.6 | 25.1 | 83.2 |
| Re/$Al_2O_3$ | Hz | 3.9 | 0.8 | 0.1 | 0.7 | 0.4 | 0.1 | 6.0 |
| Re/C | $N_2$ | 62.4 | 16.8 | 1.2 | 6.8 | 3.1 | 1.7 | 92.0 |

Table 1: Obtained yield of (2) - (7) products over Re based catalyst in MeOH under inert or hydrogen atmosphere.

| Catalyst | Solvent | Gas phase | Yield (mol%) **PRODUCTS* (2)-(7)** |
|---|---|---|---|
| Re/C | MeOH | $N_2$ | 92.0 |
| Re/C | *i*-PrOH | $N_2$ | 85.8 |
| Re/C | *n*-BuOH | $N_2$ | 81.3 |

*products are methyl, isopropyl and butyl esters of muconic, hexene dioic and adipic acid obtained in the experiment, where the temperature of 120 °C, initial 5 bar of $N_2$ and 0.2 wt% of aldaric concentration were tested.

The present disclosure relates generally to a novel catalytic process as defined in claim 1 for production of muconic and adipic acid and their esters from bio-based aldaric acids in an alcohol over a solid catalyst in an inert atmosphere (no reductive gas required). Catalyst required for this transformation consists of rhenium (Re) in its metallic form and a solid support (e.g. carbon, alumina, silica, titania), thus it is insoluble in a reaction medium.

This is the first report of an active solid catalyst for dehydroxylation of aldaric acids, which is a major benefit, as it is easy to separate from the products by a simple filtration, sedimentation and allows its use in a continuous (fixed-bed) operation.

This invention is also the first to utilize a heterogeneous (solid) catalyst and at the same time avoids the use of any corrosive reactants (e.g. HBr) and the necessity to use gaseous reductive agent (e.g. $H_2$).

The use of a simple short chain alcohol (e.g. methanol, ethanol) as a solvent shortens and simplifies the downstream (separation, purification) process, since the alcohol is volatile and the products are solid at the room temperature. In the continuous process the solvent can be easily recycled with an additional distillation unit and reused.

## Claims

**1.** Process for the production of muconic acid, adipic acid and/or their esters from aldaric acid(s), in particular from bio-based aldaric acids, comprising the following steps:

(a) feeding an aldaric acid, an alcohol and Re-catalyst on a solid support into a batch or continuous reactor, wherein the Re-catalyst is a heterogeneous catalyst comprising Re in an oxidation state 0,
(b) heating the mixture of (a) at a temperature ranging from 80 - 250 °C, preferably 100 - 140 °C, for dehydroxylation to proceed,
(c) separating the catalyst from the liquid phase, and
(d) isolating muconic acid, adipic acid and/or their esters by evaporation of residual alcohol and aldehyde/ketone and volatile short chain aldol condensation products.

**2.** The process according to claim 1, wherein heating of the mixture in step (b) is carried out for 1 - 340 h, until the conversion of hydroxylated reactant and intermediate(s) are complete, or the maximum yield of desired products is reached.

**3.** The process according to claim 1 or 2, wherein heating the mixture in step (b) is carried out with stirring, in particular at a stirring speed in a range of 100 - 10000 rpm, preferably from 300 - 2000 rpm and most preferably from 600 - 1000 rpm, preferably in a batch reactor.

**4.** The process according to any of the preceding claims, wherein the process is carried out under an inert or hydrogen atmosphere.

**5.** The process according to any of the preceding claims, wherein the temperature in step (b) is from 80-250°C, preferably 100 - 180 °C, the initial pressure of inert or hydrogen atmosphere is 0 - 500 bar, preferably 1 - 50 bar, and the time period from 0.1 to 720 h, preferably 1 to 72h.

**6.** The process according to any of the preceding claims, wherein the concentration of aldaric acid(s) in the mixture of (a) is from 0.01 to 50 wt% based on the total weight of the mixture.

**7.** The process according to any of the preceding claims, wherein the aldaric acid is selected from aldaric acids having a chain length of 6 carbon atoms (hexaric acids), in particular from the group consisting of allaric (meso), formerly

allo-mucic; galactaric (meso), formerly mucic; D- and L-glucaric (L-and D-gularic), formerly D-and L-gluco-saccharic; D- and L-mannaric, formerly D- and L-manno-saccharic; D-and L-idaric, formerly D-and L-ido-saccharic; and D- and L-altraric (D- and L-talaric), formerly D- and L-talo-mucic acid; preferably mucic acid.

**8.** The process according to any one of the preceding claims, wherein the alcohol is a short chain primary or secondary alcohol, preferably an alcohol selected from the group consisting of methanol, ethanol, 1-propanol, 2-propanol, 1-butanol and 2-butanol, most preferably methanol.

**9.** The process according to any one of the preceding claims, wherein the Re-catalyst comprises $Re^0$ on a support selected from C, $Al_2O_3$, $SiO_2$, CeOs, $TiO_2$, $CeO_2$, or $MgO_2$, preferably on neutral carbon supports.

**10.** The process according to any one of the preceding claims, wherein the Re-catalyst is bimetallic and/or wherein the mixture of (a) comprises at least one second catalyst.

**11.** The process according to claim 10, wherein the bimetallic catalyst comprises Re and at least one second metal, preferably Ru, Pd and/or Pt.

**12.** The process according to claim 10 or 11, wherein the at least one second catalyst is a hydrogenation catalyst, in particular a Pd- or Pt-catalyst.

**13.** The process according to any one of claims 10-12, wherein Re in the bimetallic catalyst, Re-catalyst in a mixture of catalysts is present in a larger amount than the second metal(s) or second catalyst(s), preferably wherein Re is present in an amount of 0.1-20 wt% relative to the support and Ru, Pd or Pt is present in an amount of 0.1-5 wt% relative to the support.

**14.** The process according to any one of the preceding claims, wherein the concentration of catalyst in the mixture of (a) ranges from 0.1 - 400 wt% based on the mass of aldaric acid.

**15.** The process according to any one of the preceding claims, wherein separating the catalyst from the liquid phase includes filtration, sedimentation or fixation of the catalyst.

**Patentansprüche**

**1.** Verfahren zur Herstellung von Muconsäure, Adipinsäure und/oder ihren Estern aus Aldarsäure(n), insbesondere aus Aldarsäuren auf biologischer Basis, umfassend die folgenden Schritte:

(a) Einspeisen einer Aldarsäure, eines Alkohols und eines Re-Katalysators auf einem festen Träger in einen Chargenreaktor oder einen kontinuierlichen Reaktor, wobei der Re-Katalysator ein heterogener Katalysator ist, der Re in einem Oxidationszustand 0 umfasst,
(b) Erhitzen des Gemischs aus (a) auf eine Temperatur im Bereich von 80 - 250°C, vorzugsweise 100 - 140°C, damit eine Dehydroxylierung erfolgt,
(c) Abtrennen des Katalysators von der flüssigen Phase und
(d) Isolieren von Muconsäure, Adipinsäure und/oder ihren Estern durch Verdampfen von restlichem Alkohol und Aldehyd/Keton-Produkten sowie flüchtigen kurzkettigen Aldol-Kondensationsprodukten.

**2.** Verfahren nach Anspruch 1, wobei das Erhitzen des Gemischs in Schritt (b) für 1 - 340 h durchgeführt wird, bis die Umwandlung des hydroxylierten Reaktanten und des Zwischenprodukts / der Zwischenprodukte vollständig ist oder die maximale Ausbeute an gewünschten Produkten erreicht ist.

**3.** Verfahren nach Anspruch 1 oder 2, wobei das Erhitzen des Gemischs in Schritt (b) unter Rühren durchgeführt wird, insbesondere mit einer Rührgeschwindigkeit im Bereich von 100 - 10000 U/min, vorzugsweise von 300 - 2000 U/min und am meisten bevorzugt von 600 - 1000 U/min, vorzugsweise in einem Chargenreaktor.

**4.** Verfahren nach einem der vorhergehenden Ansprüche, wobei das Verfahren unter einer Inert- oder Wasserstoff-Atmosphäre durchgeführt wird.

**5.** Verfahren nach einem der vorhergehenden Ansprüche, wobei die Temperatur in Schritt (b) 80 - 250 °C, vorzugsweise

100 - 180 °C, der Anfangsdruck der Inert- oder Wasserstoff-Atmosphäre 0 - 500 bar, vorzugsweise 1 - 50 bar, und die Zeitdauer 0,1 - 720 h, vorzugsweise 1 - 72 h, beträgt.

**6.** Verfahren nach einem der vorhergehenden Ansprüche, wobei die Konzentration der Aldarsäure(n) in dem Gemisch von (a) 0,01 bis 50 Gew.-% beträgt, bezogen auf das Gesamtgewicht des Gemischs.

**7.** Verfahren nach einem der vorhergehenden Ansprüche, wobei die Aldarsäure ausgewählt ist aus Aldarsäuren mit einer Kettenlänge von 6 Kohlenstoffatomen (Hexarsäuren), insbesondere ausgewählt aus der Gruppe bestehend aus Allarsäure (meso), vormals Allo-Mucinsäure; Galactarsäure (meso), vormals Mucinsäure; D- und L-Glucarsäure (L- und D-Gularsäure), vormals D- und L-Gluco-Saccharsäure; D- und L-Mannarsäure, vormals D- und L-Manno-Saccharsäure; D- und L-idarsäure, vormals D- und L-ido-Saccharsäure; und D- und L-Altrarsäure (D- und L-Talarsäure), vormals D- und L-Talo-Mucinsäure; vorzugsweise Mucinsäure.

**8.** Verfahren nach einem der vorhergehenden Ansprüche, wobei der Alkohol ein kurzkettiger primärer oder sekundärer Alkohol ist, vorzugsweise ein Alkohol ausgewählt aus der Gruppe bestehend aus Methanol, Ethanol, 1-Propanol, 2-Propanol, 1-Butanol und 2-Butanol, am meisten bevorzugt Methanol.

**9.** Verfahren nach einem der vorhergehenden Ansprüche, wobei der Re-Katalysator $Re^0$ auf einem Träger umfasst, der ausgewählt ist aus C, $Al_2O_3$, $SiO_2$, CeOs, $TiO_2$, $CeO_2$ oder $MgO_2$, vorzugsweise auf neutralen Kohlenstoffträgern.

**10.** Verfahren nach einem der vorhergehenden Ansprüche, wobei der Re-Katalysator bimetallisch ist und/oder wobei das Gemisch von (a) mindestens einen zweiten Katalysator umfasst.

**11.** Verfahren nach Anspruch 10, wobei der bimetallische Katalysator Re und mindestens ein zweites Metall, vorzugsweise Ru, Pd und/oder Pt, umfasst.

**12.** Verfahren nach Anspruch 10 oder 11, wobei der mindestens eine zweite Katalysator ein Hydrierungskatalysator, insbesondere ein Pd- oder Pt-Katalysator, ist.

**13.** Verfahren nach einem der Ansprüche 10 bis 12, wobei Re in dem bimetallischen Katalysator, bzw. der Re-Katalysator in einem Katalysatorgemisch in einer größeren Menge vorhanden ist als das (die) zweite(n) Metall(e) oder der (die) zweite(n) Katalysator(en), vorzugsweise wobei Re in einer Menge von 0,1 - 20 Gew.-%, bezogen auf den Träger, und Ru, Pd oder Pt in einer Menge von 0,1 - 5 Gew.-%, bezogen auf den Träger, vorhanden ist.

**14.** Verfahren nach einem der vorhergehenden Ansprüche, wobei die Konzentration des Katalysators in dem Gemisch aus (a) im Bereich von 0,1 - 400 Gew.-% liegt, bezogen auf die Masse der Aldarsäure.

**15.** Verfahren nach einem der vorhergehenden Ansprüche, wobei das Abtrennen des Katalysators von der flüssigen Phase eine Filtration, Sedimentation oder Fixierung des Katalysators einschließt.

**Revendications**

**1.** Procédé de production d'acide muconique, d'acide adipique et/ou de leurs esters à partir d'acide(s) aldarique(s), en particulier à partir d'acides aldariques biosourcés, comprenant les étapes suivantes :

(a) l'introduction d'un acide aldarique, d'un alcool et d'un catalyseur au Re sur un support solide dans un réacteur discontinu ou continu, dans lequel le catalyseur au Re est un catalyseur hétérogène comprenant du Re dans un état d'oxydation de 0,
(b) le chauffage du mélange de (a) à une température allant de 80 à 250 °C, de préférence de 100 à 140 °C, pour qu'une déshydroxylation se déroule,
(c) la séparation du catalyseur de la phase liquide, et
(d) l'isolement de l'acide muconique, de l'acide adipique et/ou de leurs esters par évaporation de l'alcool résiduel et des produits de condensation d'aldéhyde/cétone et d'aldol à chaîne courte volatils.

**2.** Procédé selon la revendication 1, dans lequel le chauffage du mélange dans l'étape (b) est effectué pendant 1 à 340 h, jusqu'à ce que la conversion du réactif hydroxylé et du ou des intermédiaires soit terminée, ou que le rendement maximal en produits souhaités soit atteint.

3. Procédé selon la revendication 1 ou 2, dans lequel le chauffage du mélange dans l'étape (b) est effectué sous agitation, en particulier à une vitesse d'agitation dans une plage de 100 à 10 000 tr/min, de préférence de 300 à 2 000 tr/min et de manière préférée entre toutes de 600 à 1 000 tr/min, de préférence dans un réacteur discontinu.

4. Procédé selon l'une quelconque des revendications précédentes, dans lequel le procédé est mis en oeuvre sous une atmosphère inerte ou d'hydrogène.

5. Procédé selon l'une quelconque des revendications précédentes, dans lequel la température dans l'étape (b) est de 80 à 250 °C, de préférence de 100 à 180 °C, la pression initiale de l'atmosphère inerte ou d'hydrogène est de 0 à 500 bar, de préférence de 1 à 50 bar, et la période de temps de 0,1 à 720 h, de préférence de 1 à 72 h.

6. Procédé selon l'une quelconque des revendications précédentes, dans lequel la concentration en acide(s) aldarique(s) dans le mélange de (a) est de 0,01 à 50 % en poids sur la base du poids total du mélange.

7. Procédé selon l'une quelconque des revendications précédentes, dans lequel l'acide aldarique est sélectionné parmi les acides aldariques ayant une longueur de chaîne de 6 atomes de carbone (acides hexariques), en particulier dans le groupe constitué de l'acide allarique (méso), anciennement allo-mucique ; galactarique (méso), anciennement mucique ; D- et L-glucarique (L- et D-gularique), anciennement D- et L-gluco-saccharique ; D- et L-mannarique, anciennement D- et L-manno-saccharique ; D-et L-idarique, anciennement D-et L-ido-saccharique ; et D- et L-altrarique (D- et L-talarique), anciennement D- et L-talo-mucique ; de préférence l'acide mucique.

8. Procédé selon l'une quelconque des revendications précédentes, dans lequel l'alcool est un alcool primaire ou secondaire à chaîne courte, de préférence un alcool sélectionné dans le groupe constitué du méthanol, de l'éthanol, du 1-propanol, du 2-propanol, du 1-butanol et du 2-butanol, de manière préférée entre toutes, du méthanol.

9. Procédé selon l'une quelconque des revendications précédentes, dans lequel le catalyseur au Re comprend $Re^0$ sur un support sélectionné parmi C, $Al_2O_3$, $SiO_2$, $CeO_3$, $TiO_2$, $CeO_2$ ou $MgO_2$, de préférence sur des supports neutres en carbone.

10. Procédé selon l'une quelconque des revendications précédentes, dans lequel le catalyseur au Re est bimétallique et/ou dans lequel le mélange de (a) comprend au moins un second catalyseur.

11. Procédé selon la revendication 10, dans lequel le catalyseur bimétallique comprend du Re et au moins un second métal, de préférence Ru, Pd et/ou Pt.

12. Procédé selon la revendication 10 ou 11, dans lequel l'au moins un second catalyseur est un catalyseur d'hydrogénation, en particulier un catalyseur au Pd ou au Pt.

13. Procédé selon l'une quelconque des revendications 10 à 12, dans lequel Re dans le catalyseur bimétallique, le catalyseur au Re dans un mélange de catalyseurs est présent en une plus grande quantité que le ou les seconds métaux ou le ou les seconds catalyseurs, de préférence dans lequel Re est présent en une quantité de 0,1 à 20 % en poids par rapport au support et Ru, Pd ou Pt est présent en une quantité de 0,1 à 5 % en poids par rapport au support.

14. Procédé selon l'une quelconque des revendications précédentes, dans lequel la concentration en catalyseur dans le mélange de (a) est de 0,1 à 400 % en poids sur la base de la masse d'acide aldarique.

15. Procédé selon l'une quelconque des revendications précédentes, dans lequel la séparation du catalyseur de la phase liquide comprend une filtration, une sédimentation ou une fixation du catalyseur.

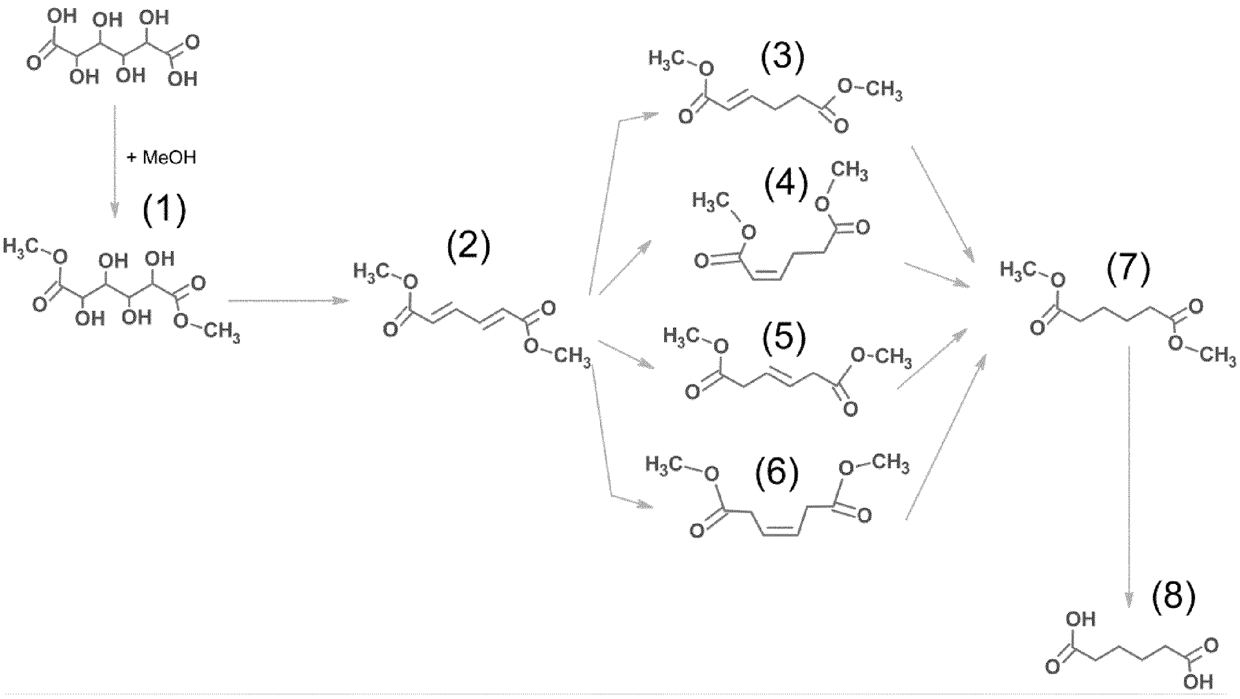


Fig.1

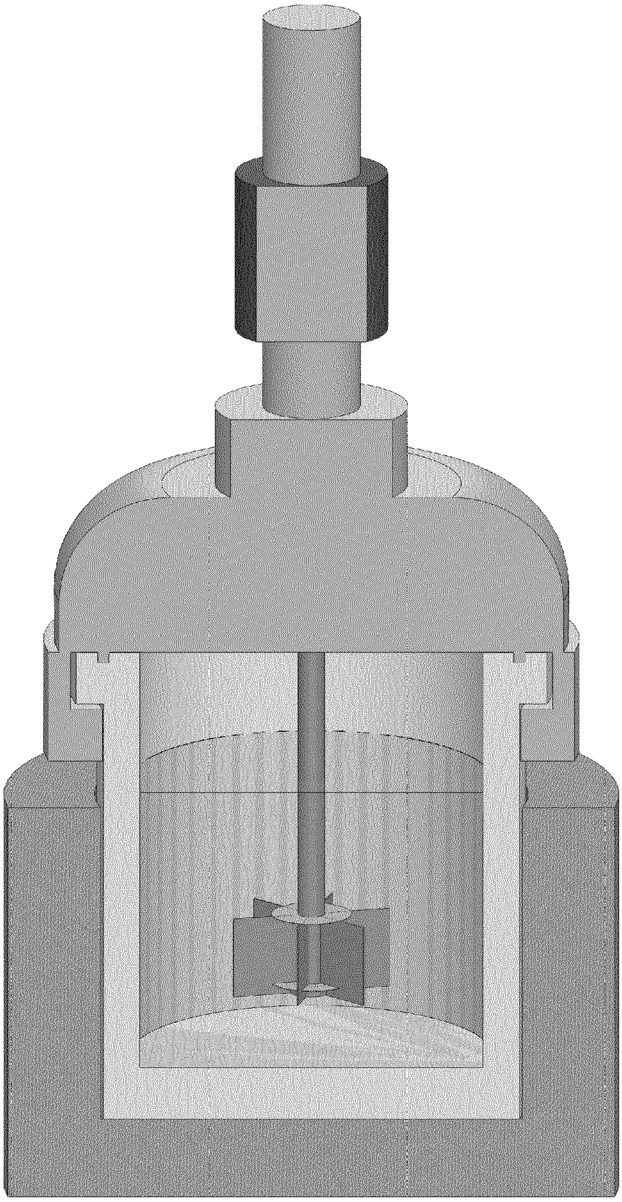

Fig. 2

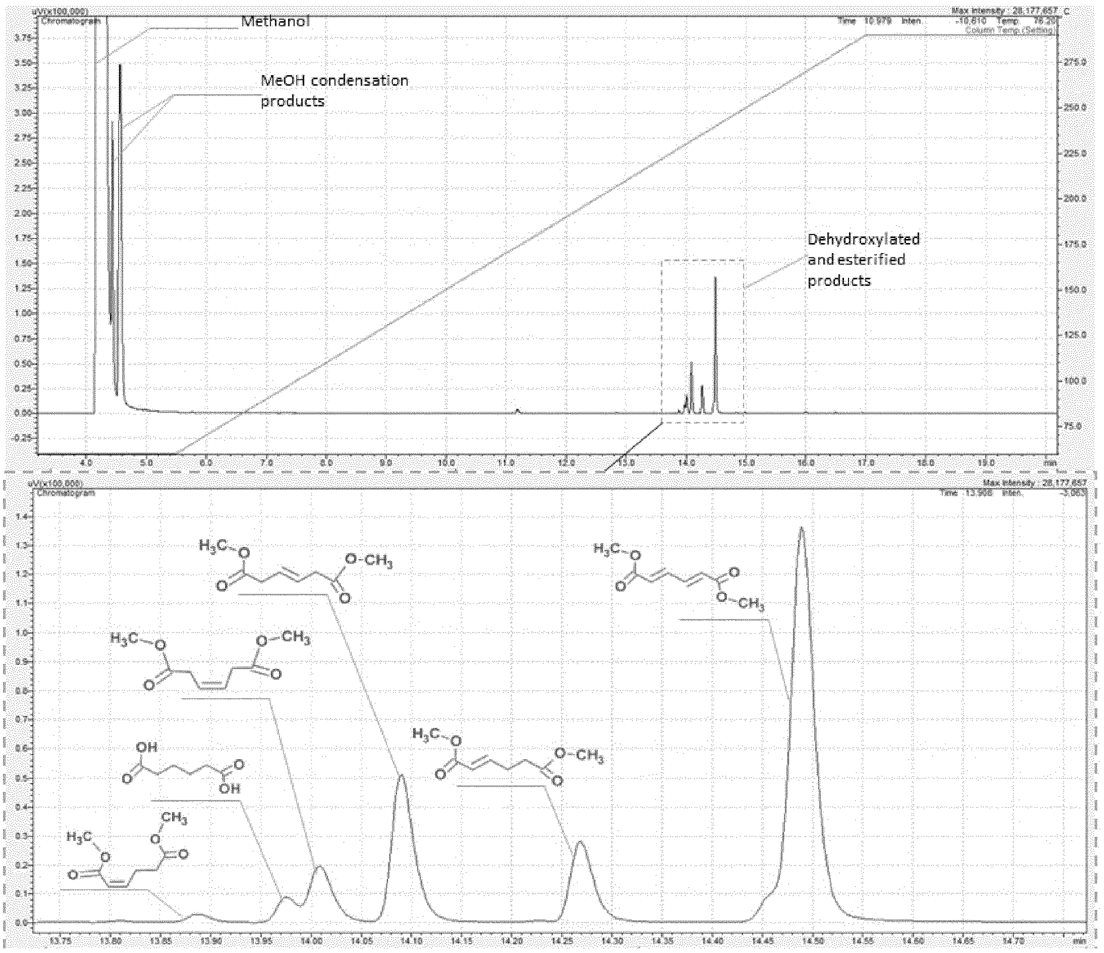
Methanol
MeOH condensation products
Dehydroxylated and esterified products

Fig. 3a

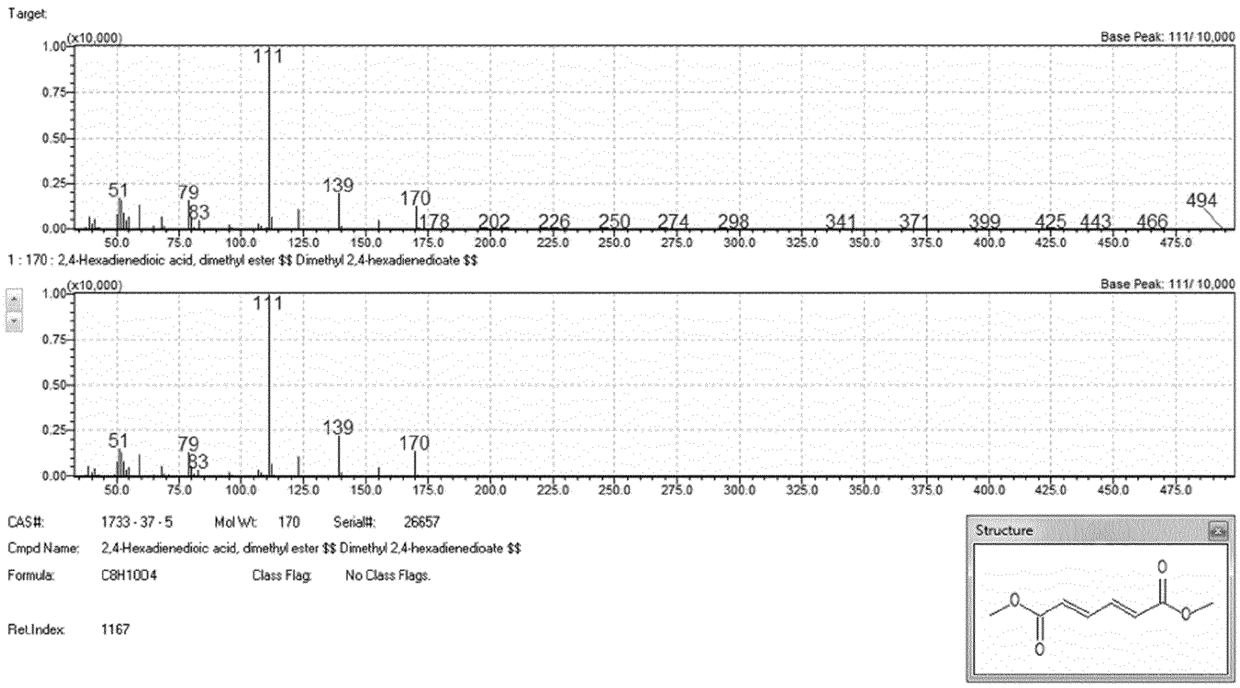
Target:
(x10,000)
Base Peak: 111/ 10,000
1.00
0.75
0.50
0.25
0.00
111
51
79
83
139
170
178
202
226
250
274
298
341
371
399
425
443
466
494
50.0 75.0 100.0 125.0 150.0 175.0 200.0 225.0 250.0 275.0 300.0 325.0 350.0 375.0 400.0 425.0 450.0 475.0
1 : 170 : 2,4-Hexadienedioic acid, dimethyl ester $$ Dimethyl 2,4-hexadienedioate $$
(x10,000)
Base Peak: 111/ 10,000
111
51
79
83
139
170
CAS#: 1733 - 37 - 5
Mol Wt: 170
Serial#: 26657
Cmpd Name: 2,4-Hexadienedioic acid, dimethyl ester $$ Dimethyl 2,4-hexadienedioate $$
Formula: C8H10O4
Class Flag: No Class Flags.
Ret.Index: 1167
Structure

Fig.3b

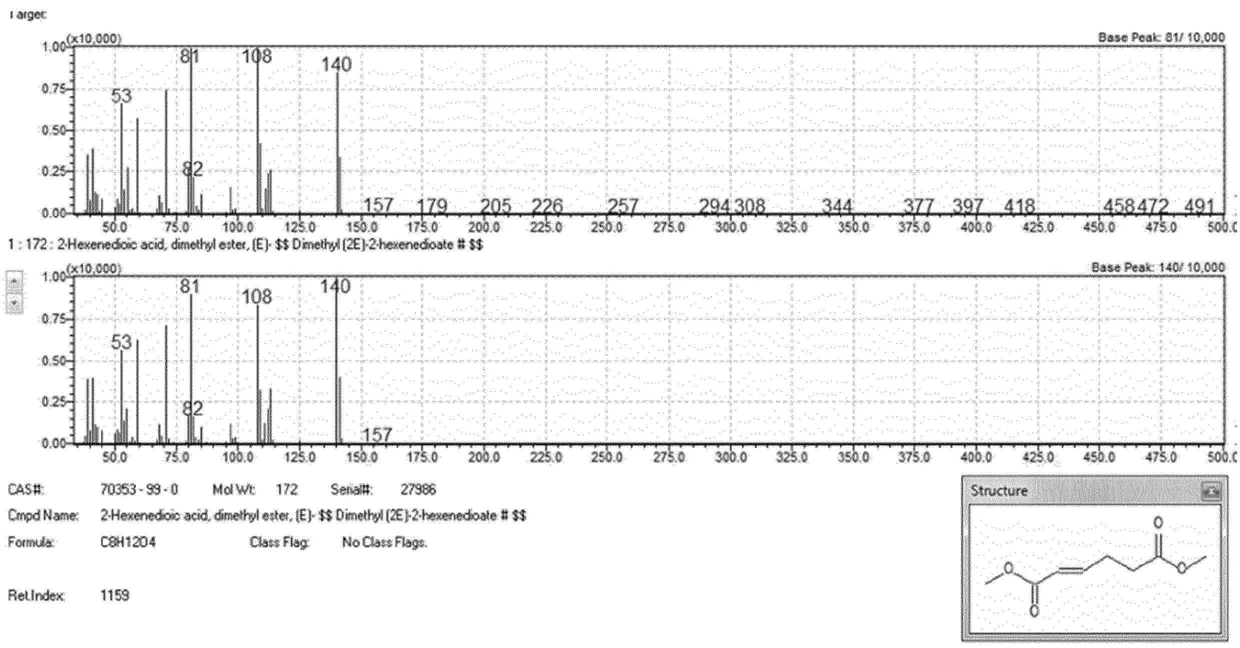
Target:
Base Peak: 81/ 10,000
1 : 172 : 2-Hexenedioic acid, dimethyl ester, (E)- $$ Dimethyl (2E)-2-hexenedioate # $$
Base Peak: 140/ 10,000
CAS#: 70353 - 99 - 0
Mol Wt: 172
Serial#: 27986
Cmpd Name: 2-Hexenedioic acid, dimethyl ester, (E)- $$ Dimethyl (2E)-2-hexenedioate # $$
Formula: C8H12O4
Class Flag: No Class Flags.
Ret.Index: 1159
Structure

Fig. 3c

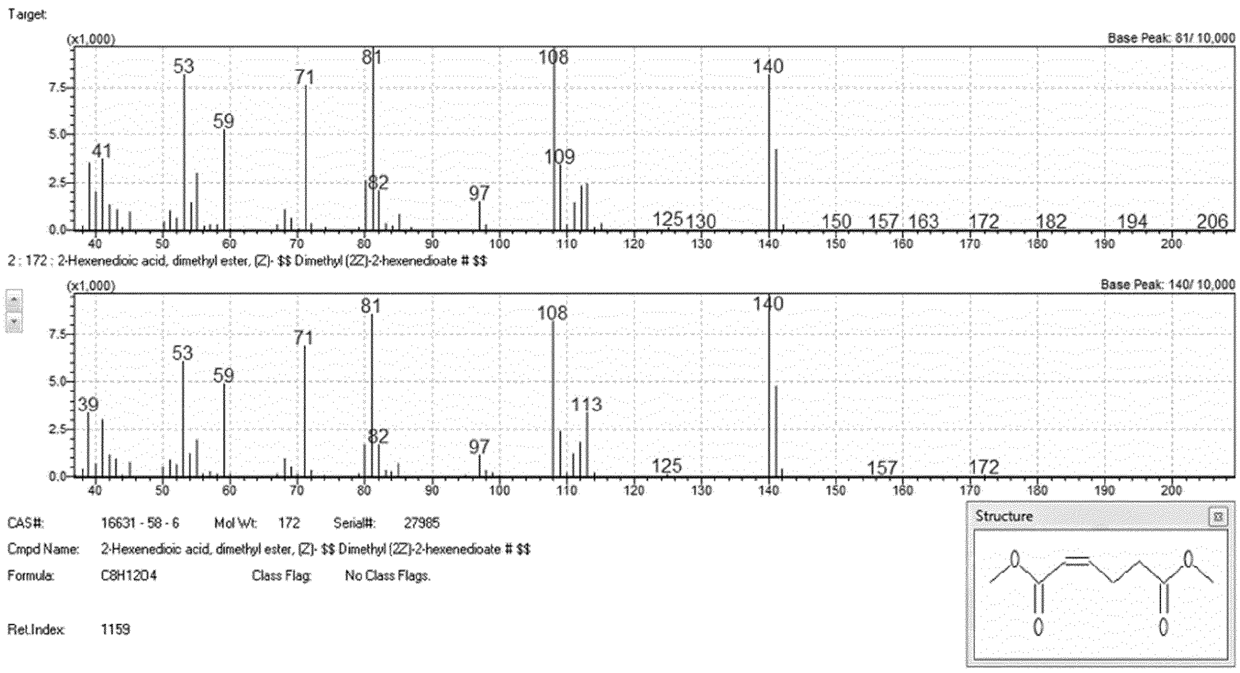
Target:
(x1,000)
Base Peak: 81/ 10,000
2 : 172 : 2-Hexenedioic acid, dimethyl ester, (Z)- $$ Dimethyl (2Z)-2-hexenedioate # $$
(x1,000)
Base Peak: 140/ 10,000
CAS#: 16631 - 58 - 6
Mol Wt: 172
Serial#: 27985
Cmpd Name: 2-Hexenedioic acid, dimethyl ester, (Z)- $$ Dimethyl (2Z)-2-hexenedioate # $$
Formula: C8H12O4
Class Flag: No Class Flags.
Ret.Index: 1159
Structure

Fig. 3d

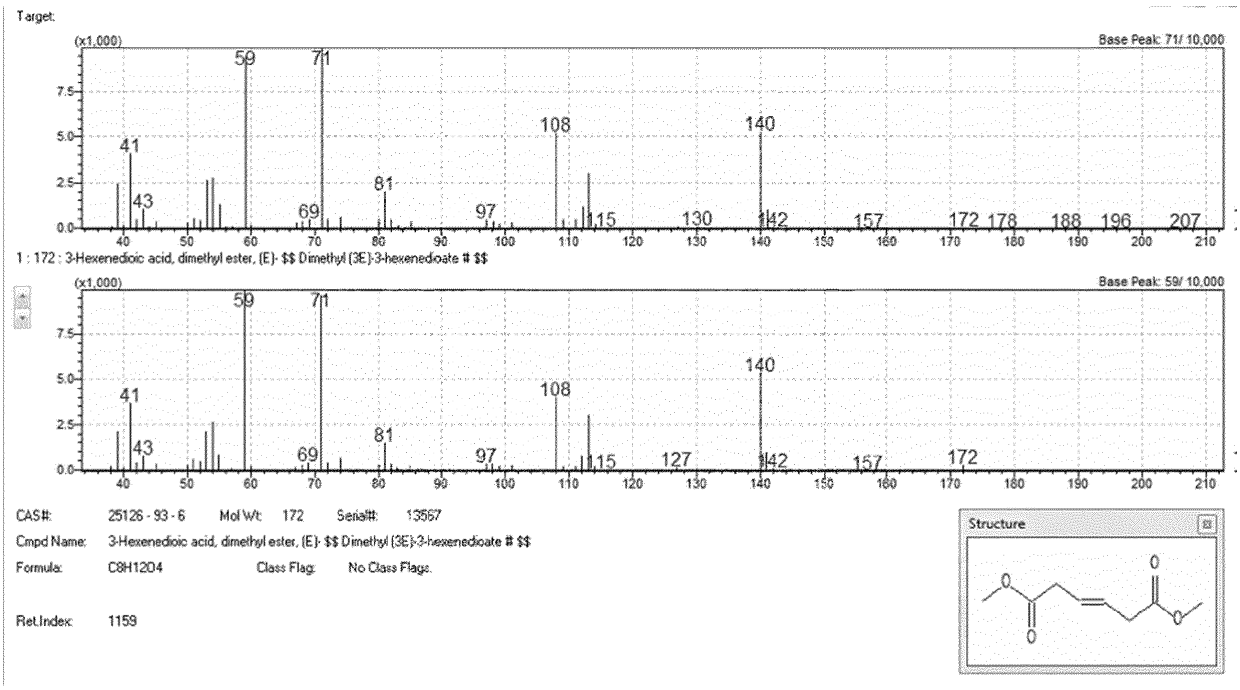
Target:
(x1,000)
Base Peak: 71/ 10,000
59
71
41
43
69
81
97
108
115
130
140
142
157
172
178
188
196
207
1 : 172 : 3-Hexenedioic acid, dimethyl ester, (E)- $$ Dimethyl (3E)-3-hexenedioate # $$
(x1,000)
Base Peak: 59/ 10,000
59
71
41
43
69
81
97
108
115
127
140
142
157
172
CAS#: 25126 - 93 - 6
Mol Wt: 172
Serial#: 13567
Cmpd Name: 3-Hexenedioic acid, dimethyl ester, (E)- $$ Dimethyl (3E)-3-hexenedioate # $$
Formula: C8H12O4
Class Flag: No Class Flags.
Ret.Index: 1159
Structure

**Fig. 3e**

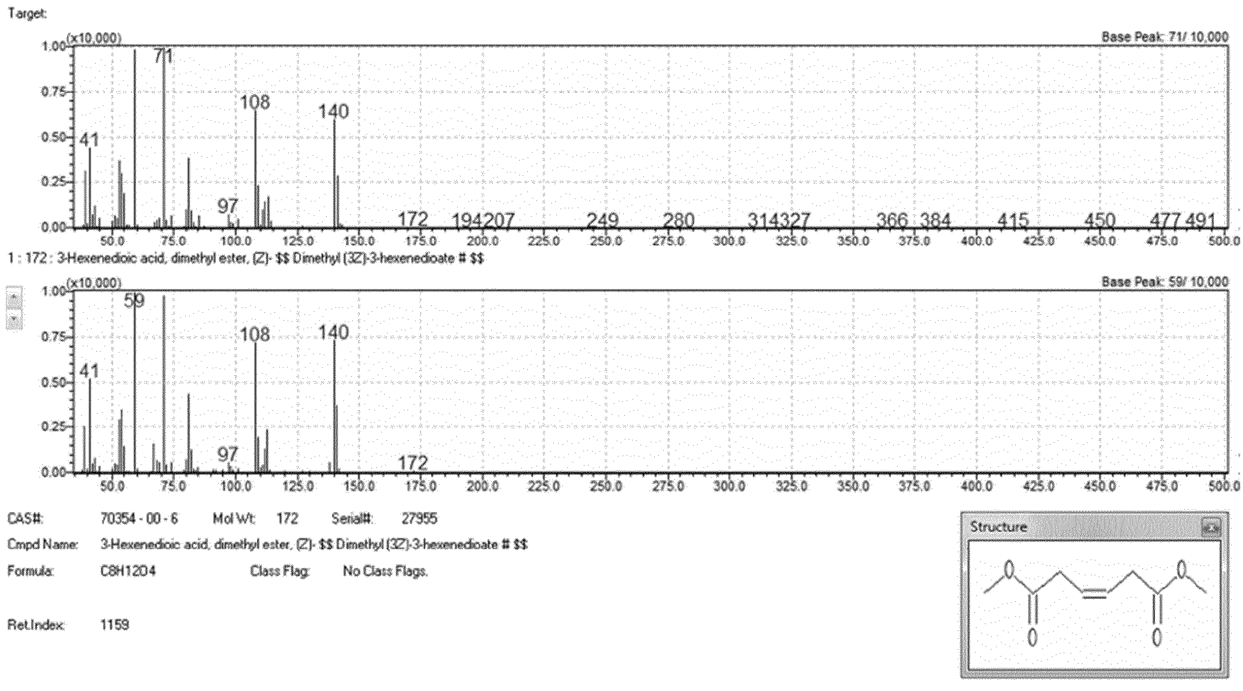
Target:
(x10,000)
Base Peak: 71/ 10,000
1.00
0.75
0.50
0.25
0.00
71
108
140
41
97
172 194207 249 280 314327 366 384 415 450 477 491
50.0 75.0 100.0 125.0 150.0 175.0 200.0 225.0 250.0 275.0 300.0 325.0 350.0 375.0 400.0 425.0 450.0 475.0 500.0
1 : 172 : 3-Hexenedioic acid, dimethyl ester, (Z)- $$ Dimethyl (3Z)-3-hexenedioate # $$
(x10,000)
Base Peak: 59/ 10,000
59
108
140
41
97
172
CAS#: 70354 - 00 - 6 Mol Wt: 172 Serial#: 27955
Cmpd Name: 3-Hexenedioic acid, dimethyl ester, (Z)- $$ Dimethyl (3Z)-3-hexenedioate # $$
Formula: C8H12O4 Class Flag: No Class Flags.
Ret.Index: 1159
Structure

**Fig. 3f**

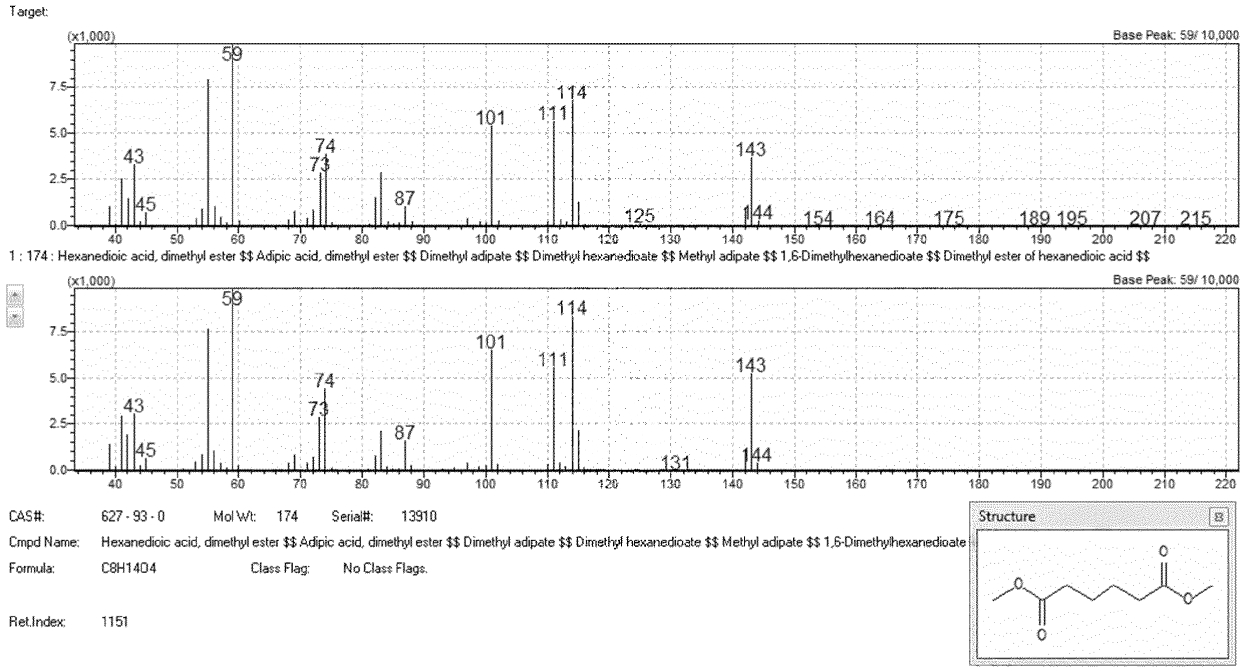
Target:
(x1,000)
Base Peak: 59/ 10,000
1 : 174 : Hexanedioic acid, dimethyl ester $$ Adipic acid, dimethyl ester $$ Dimethyl adipate $$ Dimethyl hexanedioate $$ Methyl adipate $$ 1,6-Dimethylhexanedioate $$ Dimethyl ester of hexanedioic acid $$
(x1,000)
Base Peak: 59/ 10,000
CAS#: 627 - 93 - 0
Mol Wt: 174
Serial#: 13910
Cmpd Name: Hexanedioic acid, dimethyl ester $$ Adipic acid, dimethyl ester $$ Dimethyl adipate $$ Dimethyl hexanedioate $$ Methyl adipate $$ 1,6-Dimethylhexanedioate
Formula: C8H14O4
Class Flag: No Class Flags.
Ret.Index: 1151
Structure

Fig. 3g

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FI 2015050432 W
- WO 2015189481 A
- WO 20100144862 A
- US 20100317823 A
- KR 20170016227
- WO 2016032403 A
- WO 2017207875 A


### Non-patent literature cited in the description


- *CHEMICAL ABSTRACTS,* 70197-13-6
- **LARSON et al.** *J. Am. Chem. Soc.,* 2017, vol. 139 (40), 14001-14004
- **RAJU et al.** *ACS Catal.,* 2015, vol. 5 (1), 281-300
- **SHIRAMIZO ; TOSTE.** *Angew. Chem. Int. Ed.,* vol. 52 (49), 12905-12909
- *CHEMICAL ABSTRACTS,* 10466-65-6
- *CHEMICAL ABSTRACTS,* 13768-11-1
- **LI et al.** *Angew. Chem. Int. Ed.,* vol. 53 (16), 4200-4204
- **LARSON.** *J. Am. Chem. Soc.,* 2017, vol. 139 (40), 14001-14004
- **SHIRAMIZU.** *Angew. Chem. Int. Ed.,* vol. 52 (49), 12905-12909
- **OTA et al.** *Angew. Chem. Int. Ed.,* vol. 54 (6), 1897-1900
- **OTA et al.** *ACS Catal.,* 2016, vol. 6 (5), 3213-3226
- **TAZAWA et al.** *ACS Catal.,* 2016, vol. 6 (10), 6393-6397